# FASCICULE DE BREVET EUROPEEN

(11) **EP 1 118 345 B1**
(45) Date de publication et mention de la délivrance du brevet: **15.06.2005**
(21) Numéro de dépôt: 01400026.9
(22) Date de dépôt: 08.01.2001
(51) Int. Cl.: A61M 16/01

(54) **Appareil d'anesthésie respiratoire à gestion automatique des débits gazeux**
Narkosebeatmungsgerät mit automatischer Führung der Gasflüsse
Anaesthesia respirator with automatic management of gas flow

(30) Priorité: 21.01.2000 FR 0000780
(43) Date de publication de la demande: 25.07.2001
(73) Titulaire: TAEMA, F-92182 Antony Cédex (FR)
(72) Inventeur: Kitten, Sébastien, 24130 Prigonrieux (FR)
(74) Mandataire: Pittis, Olivier

(56) Documents cités:
- EP-A- 0 697 224
- EP-A- 0 904 793
- US-A- 4 791 922

## Description

La présente invention se rapporte au domaine de l'anesthésie inhalatoire et, plus particulièrement, à un appareil d'anesthésie inhalatoire comprenant un circuit principal de gaz et des moyens permettant de gérer automatiquement les débits de gaz respiratoires pendant les phases transitoires de passage d'un mode de ventilation à un autre mode de ventilation.

Les appareils d'anesthésie comportent classiquement un système fermé de canalisations ou analogues, encore appelé circuit principal destiné, d'une part, à amener jusqu'aux voies aériennes supérieures du patient des gaz anesthésiques frais, issus d'une source de gaz anesthésiques frais et, d'autre part, de récupérer les gaz exhalés par le patient, de permettre une élimination du dioxyde de carbone contenu dans ces gaz exhalés par le patient, de permettre un recyclage et un mélange de ces gaz exhalés purifiés avec les gaz anesthésiques frais avant leur renvoi vers les voies aériennes supérieures du patient.

Ce système fermé fonctionnant en boucle permet de minimiser la quantité de gaz anesthésique frais utilisée pour anesthésier le patient.

De tels dispositifs ont déjà été décrits à maintes reprises dans l'art antérieur. Pour plus de détails, on peut se reporter notamment aux documents EP-A-643978, US-A-3,687,137, EP-A-292615 ou EP-A-266964.

Compte tenu de la diversité des situations rencontrées au cours d'une intervention chirurgicale, médicale ou analogue mettant en oeuvre une anesthésie respiratoire du patient, réalisée avec un appareil d'anesthésie respiratoire, l'opérateur de l'appareil, par exemple un médecin, un infirmier ou analogue, est contraint de modifier les modalités de la ventilation du patient, c'est-à-dire de passer successivement et indifféremment d'un premier mode dé ventilation à un deuxième mode de ventilation donné.

Habituellement, il existe trois modes de ventilation mis en oeuvre durant une opération nécessitant une anesthésie respiratoire du patient, à savoir le mode de ventilation spontanée, le mode de ventilation manuelle et le mode de ventilation mécanique.

En mode de ventilation spontanée, c'est le patient qui puise lui-même, à volonté, la quantité de gaz dont il a besoin dans un volume d'accumulation gazeux rempli d'un mélange respiratoire anesthésique. En d'autres termes, dans ce mode de ventilation, c'est le patient qui décide seul et de lui-même de l'alternance des phases inspiratoires et expiratoires durant lesquelles les gaz anesthésiques sont, respectivement, inspirés et expirés par le patient.

Par ailleurs, en mode de ventilation manuelle, la quantité de gaz respiratoire anesthésique nécessaire au patient est insufflée par un opérateur, tel un médecin ou analogue, lequel comprime manuellement un volume d'accumulation souple et compressible rempli d'un mélange anesthésique respiratoire, par exemple, un ballon de ventilation manuelle.

En revanche, en mode de ventilation mécanique, c'est l'appareil d'anesthésie respiratoire qui insuffle automatiquement la quantité de mélange anesthésique nécessaire au patient, ledit mélange anesthésique respiratoire étant préalablement accumulé et stocké dans un volume ou dispositif d'accumulation spécifique.

Pour ce faire, les appareils d'anesthésie respiratoire existants comprennent, d'une part, un volume d'accumulation spécifique dédié au mode de ventilation mécanique, par exemple un soufflet de ventilation, et, d'autre part, un autre volume d'accumulation dédié, quant à lui, au mode de ventilation manuelle et se présentant, en général, sous forme d'un ballon de ventilation manuelle.

En effet, pendant une anesthésie le praticien est amené à utiliser différents modes de ventilation impliquant l'utilisation de différents organes d'accumulation des gaz expirés par le patient.

Lors ce ces changements de mode, le circuit doit continuer à stocker un volume de gaz suffisant pour la ventilation.

Cependant, dans les systèmes d'anesthésie passifs actuels, lors du changement de mode avec passage du mode machine au mode assisté, les gaz contenus dans les organes d'accumulation ne sont pas transférés d'un organe à l'autre.

Pour un système pour un système dit "passif", l'organe d'accumulation des gaz expirés se remplit sous l'action de l'expiration du patient.

En ventilation spontanée, le ballon de ventilation manuelle n'est utilisé que pour visualiser une ventilation spontanée du malade.

Par contre, en ventilation manuelle, le ballon de ventilation manuelle sert à assister le patient par une ventilation manuelle exercée par le praticien.

Ce ballon entre en fonction dans le circuit, en lieu et place du volume d'accumulation des gaz expirés du mode machine, généralement un soufflet.

Si la commutation entre ces deux modes de ventilation s'effectue en fin de phase expiratoire, le volume d'accumulation est vide tandis que le volume d'accumulation précédemment présent est rempli par des gaz expirés.

Pour compenser ce volume de gaz mis hors circuit, le praticien utilise généralement l'oxygène rapide qui remplit le nouvel organe d'accumulation mis en jeu.

Or, la conséquence directe de cette action est une modification des concentrations établies dans son circuit par un apport en masse d'oxygène pur.

Le problème qui se pose alors est celui d'éviter une telle modification des concentrations, en particulier en gaz anesthésique, due à l'apport de l'oxygène pur, lors du passage d'une phase à l'autre.

De là, la présente invention vise à résoudre le problème suscité en proposant un appareil d'anesthésie permettant de réaliser une gestion automatique des débits de gaz frais, lors des phases transitoires de changement de mode ventilatoire, c'est-à-dire lors du passage du mode manuel au mode machine, et inversement.

Un appareil d'anesthésie respiratoire selon le préambule de la revendication 1 est connu du document EP-A-0 697 224.

La présente invention concerne alors un appareil d'anesthésie respiratoire comportant
- un circuit de gaz comprenant :
   (a) une branche inspiratoire susceptible d'acheminer un mélange de gaz anesthésique jusqu'à des moyens de liaison destinés à être reliés aux voies aériennes supérieures d'un patient ; et
   (b) une branche expiratoire susceptible d'acheminer un mélange gazeux contenant du CO₂ expiré par ledit patient, laquelle branche expiratoire comporte des moyens de purification permettant d'éliminer au moins une partie du CO₂ contenu dans ledit mélange gazeux expiré par le patient, lesdites branches inspiratoire et branche expiratoire formant un circuit en boucle,
- d'un ballon de ventilation manuelle formant un premier volume d'accumulation de gaz et susceptible d'être mis en communication fluidique avec au moins une partie dudit circuit de gaz ;
- d'une enceinte de ventilation mécanique comprenant un second volume d'accumulation de gaz susceptible d'être mis en communication fluidique avec au moins une partie dudit circuit de gaz ;
- des moyens de commutation permettant de contrôler ladite mise en communication fluidique du ballon de ventilation manuelle ou du second volume d'accumulation de l'enceinte de ventilation mécanique avec ledit circuit de gaz, pour permettre de passer d'un mode de ventilation mécanique à un mode de ventilation manuel, ou inversement,
- des moyens de détermination de phase respiratoire permettant de déterminer le début et/ou la fin d'une phase inspiratoire ou expiratoire du patient,
- des moyens de contrôle de l'apport en gaz frais, et,
- des moyens de pilotage commandant lesdits moyens de contrôle en gaz frais pour autoriser l'introduction dans le circuit de gaz d'une quantité préfixée de gaz frais, en réponse à une détermination de la fin d'au moins une phase inspiratoire du patient par lesdits moyens de détermination et après action sur lesdits moyens de commutation pour passer d'un mode de ventilation mécanique à un mode de ventilation manuelle, ou inversement.

Selon le cas, l'appareil peut comprendre l'une ou plusieurs des caractéristiques suivantes :
- l'enceinte de ventilation mécanique comporte un soufflet de ventilation.
- les moyens de commutations sont des valves électro-pneumatiques commandées par lesdits moyens de pilotage.
- que les moyens de contrôle de l'apport en gaz frais, comportent au moins une électrovanne pilotée par lesdits moyens de pilotage.
- les moyens de détermination de phase respiratoire sont un dispositif comportant au moins un capteur de pression et/ou de débit relié électriquement auxdits moyens de pilotage, ou un automate électronique permettant de fournir au moins une durée de phase de ventilation mécanique auxdits moyens de pilotage.

Le procédé de commande d'un appareil selon l'invention permet de passer d'un mode de ventilation manuelle à un mode de ventilation mécanique, dans lequel on procède selon les étapes de :
(a) commuter l'appareil en mode machine en agissant sur les moyens de commutation pour mettre en communication fluidique l'organe d'accumulation de gaz de l'enceinte de ventilation mécanique avec le circuit de gaz ;
(b) agir sur les moyens de contrôle de l'apport en gaz frais pour permettre l'introduction d'une quantité de gaz frais dans le circuit de gaz respiratoire et/ou dans l'organe d'accumulation de gaz de l'enceinte de ventilation mécanique jusqu'à obtention d'un remplissage au moins partiel de l'organe d'accumulation de gaz avec ledit gaz frais.

De plus, le procédé de commande d'un appareil selon l'invention permet de passer d'un mode de ventilation mécanique à un mode de ventilation manuel, dans lequel on procède selon les étapes de :
(a) commuter l'appareil en mode manuel en agissant sur les moyens de commutation pour mettre en communication fluidique le réservoir de ventilation manuelle avec le circuit de gaz ;
(b) détecter au moins une phase inspiratoire et/ou au moins une phase expiratoire du patient et ;
(c) agir sur les moyens de contrôle de l'apport en gaz frais pour permettre l'introduction d'une quantité de gaz frais dans le circuit de gaz respiratoire et/ou dans le réservoir de ventilation manuelle jusqu'à obtention d'un remplissage au moins partiel de l'organe de ventilation manuelle avec ledit gaz frais.

Selon le cas, les procédés de commande de l'invention peuvent comprendre l'une ou plusieurs des caractéristiques suivantes :
- on procède à un remplissage complet de l'organe de ventilation.
- on détecte au moins la fin d'une phase inspiratoire du patient par l'intermédiaire des moyens de détections de phase respiratoire.
- le gaz frais est un mélange contenant de l'azote et de l'oxygène, et éventuellement un ou plusieurs composés anesthésiques.

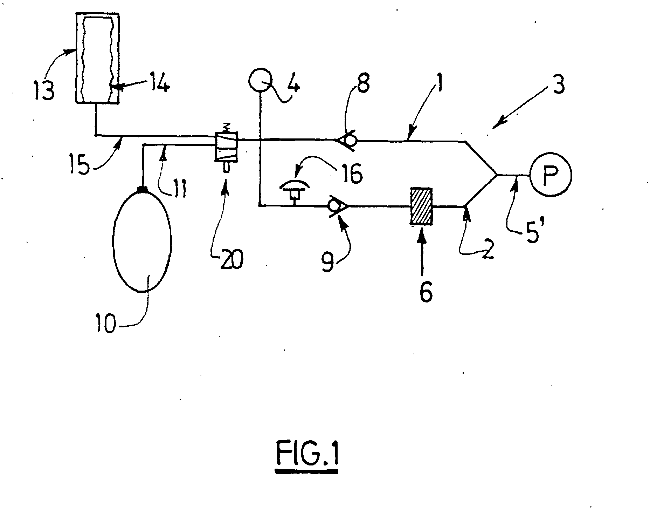

L'invention va maintenant être décrite plus en détail à l'aide de la seule figure 1 annexée représentant un mode de réalisation d'un appareil selon l'invention, donné à titre illustratif mais non limitatif.

La figure 1 représente un schéma d'un appareil d'anesthésie respiratoire selon l'invention, lequel appareil comporte un circuit de gaz en boucle 3 formé, d'une part, d'une branche inspiratoire 1 et d'une branche expiratoire 2.

La branche inspiratoire 1 achemine un gaz respiratoire anesthésique depuis une source 4 de gaz respiratoire anesthésique jusqu'à des moyens de liaisons 5 reliés aux voies aériennes supérieures d'un patient P, lesdits moyens de liaisons 5 pouvant être un masque respiratoire, une sonde d'intubation ou tout moyen analogue.

A l'inverse, la branche expiratoire 2 permet de recueillir les gaz expirés par le patient P durant les phases expiratoires, lesdits gaz expirés étant riches en dioxyde de carbone (CO₂) et contenant des produits anesthésiques qu'il est souhaitable de pouvoir récupérer et recycler en vu de leur renvoi ultérieur vers le patient P.

Lesdites branches inspiratoires 1 et expiratoires 2 forment un circuit en boucle 3, encore appelé circuit fermé.

La branche inspiratoire 1 ou la branche expiratoire 2 est équipée d'une valve 16 d'échappement à l'atmosphère permettant de palier toute surpression au sein de ladite. branche inspiratoire, laquelle surpression pourrait être néfaste pour le patient P.

Par ailleurs, la branche expiratoire 2 comporte des moyens de purification 6, tel un dispositif d'épuration contenant un adsorbant, par exemple, de la chaux ou analogue, destiné à éliminer la majeure partie du CO₂ contenu dans les gaz expirés par le patient.

En outre, la branche inspiratoire 1 et la branche expiratoire 2 sont équipées de clapets ou valves anti-retour 8 et 9, respectivement.

L'appareil d'anesthésie de la figure 1 comprend, par ailleurs, un ballon de ventilation manuelle 10 formant un volume d'accumulation de gaz, lequel est relié via une ligne 11, au circuit de gaz en boucle 3, en aval du dispositif d'épuration 6 destiné à éliminer le CO₂ des gaz expirés par le patient P.

En outre, l'appareil d'anesthésie respiratoire comporte une enceinte 13 de ventilation mécanique, au sein de laquelle est inséré un soufflet 14 de ventilation mécanique qui est relié au circuit de gaz 3, via une ligne 15.

Des moyens de commutation 20 sont agencés entre le circuit 3 de gaz et lesdites lignes 11 et 15 de manière à contrôler les circulations de gaz entre, d'une part, le ballon de ventilation manuelle 10 et le soufflet 14, et, d'autre part, ledit circuit 3 de gaz, et inversement.

Selon la présente invention, lors d'un basculement mode manuel/mode machine, on augmente le débit de gaz frais et on envoie un volume de gaz déterminé, soit le volume de l'accumulateur machine, dans le circuit 3 pour remplir le nouvel accumulateur mis en jeu, c'est-à-dire le soufflet 14.

A l'inverse, dans le cas d'un basculement mode machine/mode manuel, le ventilateur attend la fin de l'insufflation en mode automatique pour basculer vers le mode manuel, de manière à vider et remplacer le soufflet 14 par l'accumulateur 10 manuel et remplir ce dernier par, d'une part, par les gaz expirés par le patient et, d'autre part, par des gaz frais introduits dans le circuit 3 ou des gaz résiduels contenus dans le soufflet 14. En d'autres termes, les deux accumulateurs 10 et 14 sont mis en communication, afin de transvaser le gaz restant dans le soufflet 14 après l'insufflation vers l'accumulateur 10 manuel.

Toutes ces commutations d'un mode ventilatoire à un autre mode ventilatoire sont gérées électroniquement grâce à des vannes pilotées par un ensemble à microprocesseur lors de chaque détection d'une consigne de changement de mode de ventilation.

## Revendications

1. Appareil d'anesthésie respiratoire comportant
- un circuit de gaz (1,2,3) comprenant :
(a) une branche inspiratoire (1) susceptible d'acheminer un mélange de gaz anesthésique jusqu'à des moyens de liaison (5) destinés à être reliés aux voies aériennes supérieures d'un patient ; et
(b) une branche expiratoire (2) susceptible d'acheminer un mélange gazeux contenant du CO₂ expiré par ledit patient, laquelle branche expiratoire (2) comporte des moyens de purification (6) permettant d'éliminer au moins une partie du CO₂ contenu dans ledit mélange gazeux expiré par le patient, lesdites branches inspiratoire (1) et branche expiratoire (2) formant un circuit en boucle (3),
- d'un ballon (10) de ventilation manuelle formant un premier volume d'accumulation de gaz et susceptible d'être mis en communication fluidique avec au moins une partie dudit circuit de gaz (1,2,3) ;
- d'une enceinte (13) de ventilation mécanique comprenant un second volume d'accumulation (14) de gaz susceptible d'être mis en communication fluidique avec au moins une partie dudit circuit de gaz (1,2,3) ;
- des moyens de commutation (20) permettant de contrôler ladite mise en communication fluidique du ballon (10) de ventilation manuelle ou du second volume d'accumulation (14) de l'enceinte (13) de ventilation mécanique avec ledit circuit de gaz (1,2,3), pour permettre de passer d'un mode de ventilation mécanique à un mode de ventilation manuel, ou inversement
**caractérisé en ce que**
- des moyens de détermination de phase respiratoire permettant de déterminer le début et/ou la fin d'une phase inspiratoire ou expiratoire du patient,
- des moyens de contrôle de l'apport en gaz frais, et,
- des moyens de pilotage commandant lesdits moyens de contrôle en gaz frais pour autoriser l'introduction dans le circuit de gaz (1,2,3) d'une quantité préfixée de gaz frais, en réponse à une détermination de la fin d'au moins une phase inspiratoire du patient par lesdits moyens de détermination et après action sur lesdits moyens de commutation (20) pour passer d'un mode de ventilation mécanique à un mode de ventilation manuelle, où inversement.

2. Appareil selon la revendication 1, **caractérisé en ce que** l'enceinte (13) de ventilation mécanique comporte un soufflet (14) de ventilation.

3. Appareil selon l'une des revendications 1 ou 2, **caractérisé en ce que** les moyens de commutations (20) sont des valves électro-pneumatiques commandées par lesdits moyens de pilotage.

4. Appareil selon l'une des revendications 1 ou 2, **caractérisé en ce que** les moyens de contrôle de l'apport en gaz frais, comportent au moins une électrovanne pilotée par lesdits moyens de pilotage.

5. Appareil selon l'une des revendications 1 ou 2, **caractérisé en ce que** les moyens de détermination de phase respiratoire sont un dispositif comportant au moins un capteur de pression et/ou de débit relié électriquement auxdits moyens de pilotage, ou un automate électronique permettant de fournir au moins une durée de phase de ventilation mécanique auxdits moyens de pilotage.

## Patentansprüche

1. Narkosebeatmungsgerät, das Folgendes umfasst.
- einen Gaskreislauf (1, 2, 3), der Folgendes aufweist:
(a) einen Einatmezweig (1), der ein Narkosegasgemisch bis zu Verbindungsmitteln (5) befördern kann, die mit den oberen Luftwegen eines Patienten verbunden werden sollen; und
(b) einen Ausatmezweig (2), der ein von dem Patienten ausgeatmetes CO₂ enthaltendes Gasgemisch befördern kann und Reinigungsmittel (6) aufweist, die es gestatten, zumindest einen Teil des in dem von dem Patienten ausgeatmeten Gasgemisch enthaltenen CO₂ zu beseitigen, wobei der Einatmezweig (1) und der Ausatmezweig (2) einen geschlossenen Kreislauf (3) bilden,
- einen Ballon (10) zur manuellen Ventilation, der ein erstes Gasspeichervolumen bildet und mit mindestens einem Teil des Gaskreislaufs (1, 2, 3) in Strömungsverbindung gesetzt werden kann;
- einen Raum (13) zur mechanischen Ventilation, der ein zweites Gasspeichervolumen (14) enthält, das mit mindestens einem Teil des Gaskreislaufs (1, 2, 3) in Strömungsverbindung gesetzt werden kann;
- Schaltmittel (20), die es gestatten, die Herstellung der Strömungsverbindung des Ballons (10) zur manuellen Ventilation oder des zweiten Speichervolumens (14) des Raums (13) zur mechanischen Ventilation mit dem Gaskreislauf (1, 2, 3) zu steuern, um einen Wechsel von dem Modus der mechanischen Ventilation zum Modus der manuellen Ventilation oder umgekehrt zu gestatten,
**gekennzeichnet durch**
- Mittel zur Bestimmung der Atmungsphase, die das Bestimmen des Durchflusses und/oder des Endes einer Einatme- oder Ausatmephase des Patienten gestatten,
- Mittel zur Steuerung der Frischgasversorgung und
- Bedienmittel, die die Frischgassteuermittel betätigen, um das Einleiten einer vorbestimmten Frischgasmenge in den Gaskreislauf (1, 2, 3) als Reaktion auf ein Bestimmen des Endes zumindest einer Einatmephase des Patienten **durch** die Bestimmungsmittel und nach Einwirken auf die Schaltmittel (20) zum Wechsel von einem Modus der mechanischen Ventilation zu einem Modus der manuellen Ventilation oder umgekehrt, zu gestatten.

2. Gerät nach Anspruch 1, **dadurch gekennzeichnet, dass** der Raum (13) zur mechanischen Ventilation einen Ventilationsbalg (14) aufweist.

3. Gerät nach Anspruch 1 oder 2, **dadurch gekennzeichnet, dass** die Schaltmittel (20) elektropneumatische Ventile sind, die durch die Bedienmittel betätigt werden.

4. Gerät nach Anspruch 1 oder 2, **dadurch gekennzeichnet, dass** die Mittel zur Steuerung der Frischgasversorgung mindestens ein Magnetventil umfassen, das durch die Bedienmittel betätigt wird.

5. Gerät nach Anspruch 1 oder 2, **dadurch gekennzeichnet, dass** die Mittel zur Bestimmung der Atmungsphase eine mindestens einen elektrisch mit den Bedienmitteln verbundenen Druck- und/oder Durchflusssensor enthaltende Vorrichtung oder eine elektronische automatische Einrichtung, die den Bedienmitteln mindestens eine mechanische Ventilationsphase liefert, sind.

## Claims

1. Respiratory anaesthesia apparatus with:
- a gas circuit (1, 2, 3) comprising:
(a) an inhalation branch (1) which can convey a mixture of anaesthetic gas to connection means (5) which are intended to be connected to the upper airways of a patient; and
(b) an exhalation branch (2) which can convey a gaseous mixture containing CO₂ exhaled by said patient, which exhalation branch (2) includes purification means (6) with which it is possible to eliminate at least some of the CO₂ contained in said gaseous mixture exhaled by the patient, said inhalation branch (1) and said exhalation branch (2) forming a loop circuit (3),
- a manual ventilation balloon (10) forming a first gas accumulation volume and capable of being brought into fluid communication with at least part of said gas circuit (1, 2, 3);
- a mechanical ventilation enclosure (13) comprising a second gas accumulation volume (14) capable of being brought into fluid communication with at least part of said gas circuit (1, 2, 3);
- switching means (20) with which it is possible to control said fluid communication of the manual ventilation balloon (10) or of the second accumulation volume (14) of the mechanical ventilation enclosure (13) with said gas circuit (1, 2, 3), to permit change-over from a mechanical ventilation mode to a manual ventilation mode, or vice versa,
- means for determining the respiratory phase, making it possible to determine the start and/or the end of an inhalation or exhalation phase of the patient, **characterized by**:
- means for controlling the supply of fresh gas, and
- actuating means controlling said control means for fresh gas in order to permit introduction of a pre-defined quantity of fresh gas, into the gas circuit (1, 2, 3), in response to determination of the end of at least one inhalation phase of the patient by said determination means and after acting on said switching means (20) to change over from a mechanical ventilation mode to a manual ventilation mode, or vice versa.

2. Apparatus according to Claim 1, **characterized in that** the mechanical ventilation enclosure (13) includes a ventilation bellows (14).

3. Apparatus according to either of Claims 1 and 2, **characterized in that** the switching means (20) are electropneumatic valves controlled by said actuating means.

4. Apparatus according to either of Claims 1 and 2, **characterized in that** the means for controlling the supply of fresh gas include at least one solenoid valve controlled by said actuating means.

5. Apparatus according to either of Claims 1 and 2, **characterized in that** the means for determination of respiratory phase are a device including at least one pressure sensor and/or flowrate sensor connected electrically to said actuating means, or an electronic automatic member with which it is possible to supply at least one mechanical ventilation phase duration to said actuating means.
